# EUROPÄISCHE PATENTSCHRIFT

(11) **EP 0 765 855 B1**
(45) Veröffentlichungstag und Bekanntmachung des Hinweises auf die Patenterteilung: **06.10.1999**
(21) Anmeldenummer: 96112071.4
(22) Anmeldetag: 26.07.1996
(51) Int. Cl.: C07C 51/50, C08G 69/36

(54) **Verfahren zur Herstellung von Copolyamiden**
Process for the preparation of colpolyamides
Procédé de préparation de copolyamides

(30) Priorität: 28.09.1995 DE 19536056
(43) Veröffentlichungstag der Anmeldung: 02.04.1997
(73) Patentinhaber: HÜLS AKTIENGESELLSCHAFT, 45764 Marl (DE)
(72) Erfinder: Mumcu, Salih, Dr., 45772 Marl (DE); Baumann, Franz-Erich, Dr., 48249 Dülmen (DE)

(56) Entgegenhaltungen:
- EP-A- 0 313 436
- DE-A- 1 745 465
- DE-A- 1 745 466
- DE-A- 2 633 110
- PATENT ABSTRACTS OF JAPAN vol. 002, no. 052 (C-011), 14.April 1978 & JP-A-53 010693 (MITSUBISHI CHEM IND LTD), 31.Januar 1978,

## Beschreibung

Gegenstand der Erfindung ist ein Verfahren zur Herstellung von Copolyamiden unter Verwendung flüssiger Lösungen von Dicarbonsäuren mit 6 bis 44 C-Atomen im Kohlenstoffgerüst.

Ein breites Anwendungsgebiet für Dicarbonsäuren ist z. B. ihr Einsatz als Rohstoff zur Herstellung von Polyamiden. Ein wohlfeiles Herstellungsverfahren, das die bei der konventionellen Vorgehensweise über Nylonsalzlösungen erforderlichen Wassermengen eliminiert, sollte von aufgeschmolzenen Diaminen und Dicarbonsäuren ausgehen. Hierbei werden die Komponenten aufgeschmolzen, vermischt und in der Schmelze polykondensiert. In der Praxis liegt ein wesentlicher Nachteil für das Verfahren darin, daß die Dicarbonsäure nicht ohne Verfärbung aufgeschmolzen werden kann und sich in geschmolzenem Zustand bei längerer Lagerung stark verfärbt. Diese Verfärbung wird dann auf das auskondensierte Polyamid übertragen. Ein derartiges Produkt wird auf dem Markt nicht akzeptiert. Aromatische Dicarbonsäuren, z. B. Terephthalsäure, können unzersetzt gar nicht aufgeschmolzen werden.

In der Praxis weicht man daher auf eine Polykondensation in wäßriger Phase aus. Dieses Verfahren verlangt, daß im technischen Maßstab große Mengen an Wasser erhitzt und abgedampft werden müssen (EP-A-0 122 005).

Aus diesen Gründen ist es wünschenswert, praxisgerechte Wege zu finden, die Polykondensation in der Schmelze durchzuführen.

In der EP-A-0 313 436, der DE-A-1 745 465 und der DE-A-1 745 466 werden transparente Copolyamide beschrieben, die durch Vorreaktion von Lactam und Dicarbonsäure bei hohen Temperaturen und anschließende Reaktion des Produkts mit Diamin erhalten werden können. Die Komponenten werden dort als solche zugegeben und zusammen aufgeheizt.

In der DE-A-2 633 110 wird beschrieben, daß geringe Verunreinigungen in Dicarbonsäuren und Diaminen mit Hilfe von Hydrazinhydrat unschädlich gemacht werden können, so daß die daraus hergestellten Salze bzw. Polyamide eine verbesserte Farbqualität besitzen.

Die JP-A-53 010 693 offenbart, daß bei der Herstellung von Copolyamiden nach der Zugabe einer bestimmten Lactammenge zur wäßrigen Lösung eines Nylonsalzes diese konzentriert werden kann, ohne daß das Nylonsalz polymerisiert.

Die Aufgabe der Erfindung bestand darin, bei einem Verfahren zur Herstellung von Copolyamiden eine schmelzeflüssige Dicarbonsäure einzusetzen, die auch bei längerer Lagerung keine Verfärbung zeigt.

Das aufgezeigte Problem wird überwunden, indem die Carbonsäure in einem Lactam einer aliphatischen α, ω-Aminocarbonsäure mit 6 bis 16 C-Atomen im Kohlenstoffgerüst gelöst wird.

Als Lactame kommen solche infrage, die von einer aliphatischen α, ω-Aminocarbonsäure mit 6 bis 16, vorzugsweise 6 bis 12, C-Atomen im Kohlenstoffgerüst abgeleitet sind. Insbesondere seien Caprolactam, Laurinlactam, Capryllactam, Caprinlactam, Oenanthlactam und Pelargonlactam genannt. Es können auch Gemische von Lactamen eingesetzt werden.

Als Dicarbonsäuren können aliphatische, cycloaliphatische sowie aromatische Dicarbonsäuren mit 6 bis 44, vorzugsweise 6 bis 13, C-Atomen im Kohlenstoffgerüst eingesetzt werden. Als Beispiele seien Adipin-, Kork-, Azelain-, Sebacin-, Undecandicarbon-, Dodecandicarbonsäure, dimerisierte Fettsäuren, Brassylsäure, Cyclohexandicarbonsäure, Terephthalsäure, Isophthalsäure, 2,6-Naphthalindicarbonsäure und/oder 4,4'-Biphenyldicarbonsäure genannt.

Die Herstellung der erfindungsgemäßen flüssigen Dicarbonsäure-Lösungen erfolgt im allgemeinen durch Zugabe der festen Dicarbonsäure ins flüssige Lactam, bis eine Klare Lösung erhalten worden ist. Die Herstellung beschränkt sich aber nicht auf die angeführte Arbeitsmethode. Jede andere technisch sinnvolle Arbeitsweise kann mit gleichem Erfolg angewendet werden.

Es ist möglich, bis zum 4-fachen, vorzugsweise bis zum 1,5-fachen - bezogen auf die Lactammasse - an Dicarbonsäure in dem Lactam zu lösen.

Für die Lagerung wird eine möglichst tiefe Temperatur angestrebt. Als vorteilhaft haben sich Temperaturen im Bereich von 60 bis 160 °C, vorzugsweise von 100 bis 140 °C, erwiesen.

Bei der Herstellung von Copolyamiden können größere Mengen Lösung über mehrere Tage flüssig gelagert werden ohne daß in der Lösung oder bei dem daraus hergestellten Copolyamid Verfärbungen auftreten. Gleichzeitig konnte überraschend festgestellt werden, daß das in der Lösung enthaltene Lactam - selbst wenn Anteile von Wasser enthalten sind - keine Neigung zeigt, unlösliche Polykondensate zu bilden.

Bei der Verwendung der anspruchsgemäßen Lösungen als Ausgangsmaterial für die Copolyamid-Herstellung empfiehlt es sich, ein Dicarbonsäure/Lactam-Mengenverhältnis einzustellen, wie es für das Copolyamid erforderlich ist.

Die genannten Parameter werden mit Hilfe nachstehender Meßverfahren bestimmt.

Die **Bestimmung der Lösungsviskosität** (rel. Viskositätszahl ηᵣₑₗ) **der Copolyamide** erfolgt unter Verwendung einer 0,5 gew.-%igen m-Kresol-Lösung bei 25 °C (DIN 53 727 / ISO 307).

Die **Bestimmung der Schmelztemperatur** wird mittels DSC durchgeführt (ASTM D 3408).

Die **Bestimmung der Farbzahl gemäß APHA** erfolgt in Küvetten (Höhe: 300 mm - Durchmesser: 25 mm) an einer methanolischen Lösung (20 Gew.-%) im Tageslicht, wobei diese Lösung gegen wässrige Lösungen von CoCl₂ und K₂PtCl₆ in verschiedenen Konzentrationen als Farbstandards verglichen werden (DIN 53 409).

Mit Buchstaben gekennzeichnete Beispiele sind nicht erfindungsgemäß.

### Beispiele

### Beispiel A:

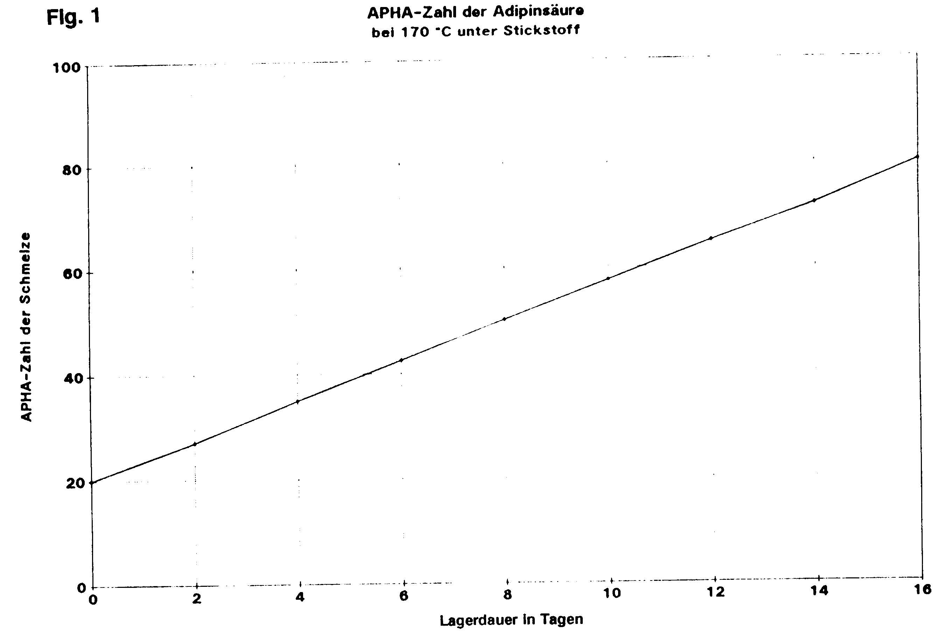

2,5 kg Adipinsäure werden in einem 5 l-Stahlautoklaven mit Blattrührer und Bodenventil entgast und dann unter einer N₂-Atmosphäre auf 170 °C erhitzt. Alle 2 Tage werden Proben entnommen; die Zunahme der APHA-Farbzahl über 16 Tage geht aus der Fig. 1 hervor.

### Beispiel 1:

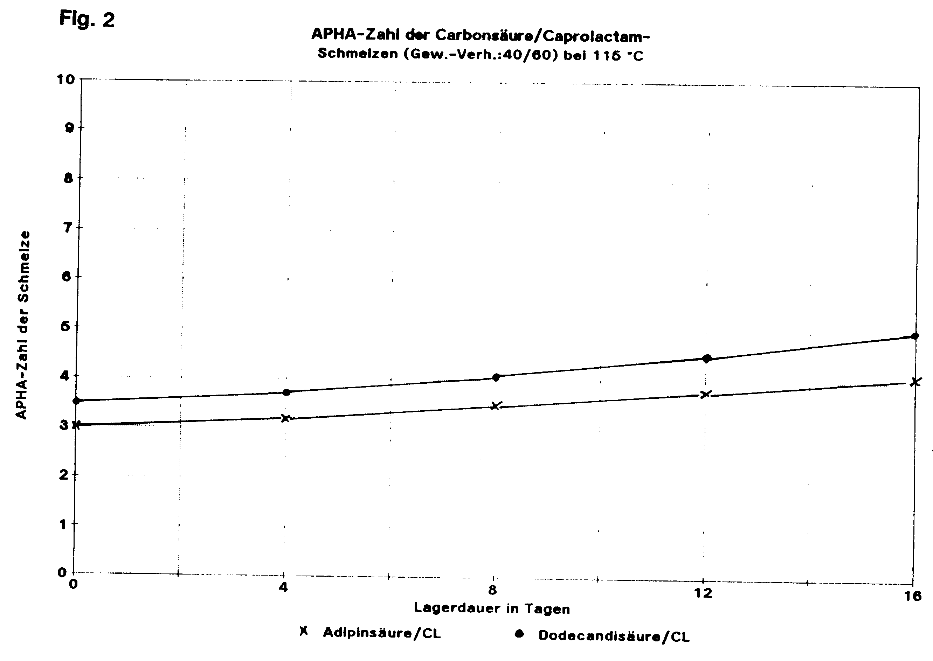

Eine Mischung aus 40 Gew.-% Adipinsäure und 60 Gew.-% Caprolactam wird wie im Beispiel A, aber bei 115 °C, behandelt. Bei dieser Temperatur ist die Mischung vollkommen aufgeschmolzen. Die Farbzahlveränderung über 16 Tage geht aus der Fig. 2 hervor.

### Beispiel B:

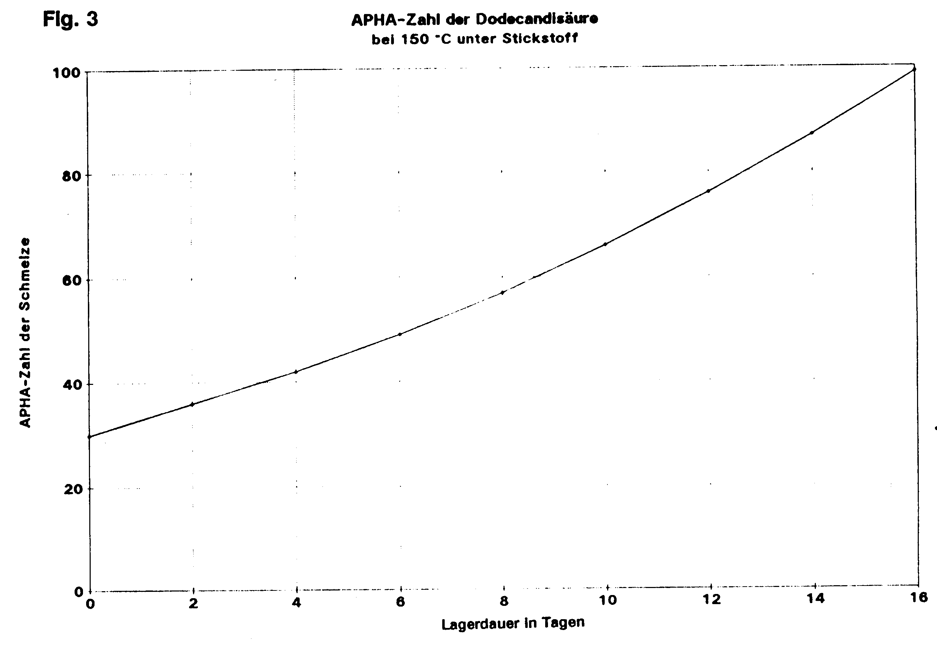

Entsprechend dem Beispiel A beläßt man Dodecandisäure 16 Tage bei 150 °C. Die Fig. 3 verdeutlicht die Zunahme der APHA-Farbzahl während dieses Zeitraumes.

### Beispiel 2:

Das Beispiel 1 wird mit einer Mischung von 40 Gew.-% Dodecandisäure und 60 Gew.-% Caprolactam wiederholt; siehe Fig. 2.

### Beispiel C:

49,50 kg Laurinlactam werden in einem Polykondensationskessel vorgelegt. Dann werden aus einem bei 150 °C Lagertemperatur gehaltenen Vorratstank 28,89 kg Dodecandisäure in den Polykondensationskessel gedrückt. Gleichzeitig werden 21,61 kg 1,10-Decandiamin in einem Aufschmelzkessel unter Stickstoff auf 80 °C erwärmt, 2 h verrührt und über ein 90 µm-Filter in den Polykondensationskessel gedrückt. Danach heizt man unter Rühren innerhalb von 3 h auf 235 °C auf, beläßt den Ansatz 1 h bei dieser Temperatur und bringt dann unter kontinuierlicher Druckentspannung die Schmelze auf 280 °C. Man entspannt innerhalb von 1,5 h auf Atmosphärendruck, leitet ½ h Stickstoff über die Schmelze und fährt diese dann als Stranggranulat aus. Man erhält 93,60 kg honiggelbes Granulat; ηᵣₑₗ = 1,79; Schmelzpunkt: 163 °C.

Aus diesem Granulat wird eine 80 µm-Flachfolie extrudiert. Diese zeigt eine gelbe Eigenfarbe, Schlieren und Stippen.

### Beispiel 3:

In einem Lagertank wird bei 115 °C ein Gemisch aus 63,15 Gew.-Teilen Laurinlactam und 36,85 Gew.-Teilen Dodecandisäure bevorratet. Von diesem Gemisch werden 78,386 kg in einen Polykondensationskessel gedrückt. In einem Aufschmelzkessel werden 21,613 kg 1,10-Decandiamin unter Stickstoff auf 80 °C erwärmt und 2 h verrührt. Dann wird wie im Beispiel C weiter vorgegangen. Man erhält 93,57 kg farbloses Granulat, ηᵣₑₗ = 1,86; Schmelzpunkt: 165 °C.

Die daraus extrudierte 80 µm-Flachfolie ist farblos und frei von Schlieren und Stippen.

## Patentansprüche

1. Verfahren zur Herstellung von Copolyamiden,
dadurch gekennzeichnet,
daß hierzu die flüssige Lösung einer Dicarbonsäure mit 6 bis 44 C-Atomen im Kohlenstoffgerüst in einem Lactam einer aliphatischen α, ω-Aminocarbonsäure mit 6 bis 16 C-Atomen im Kohlenstoffgerüst eingesetzt wird, wobei die flüssige Lösung bei einer Temperatur im Bereich von 60 bis 160 °C bevorratet wird.

2. Verfahren gemäß Anspruch 1,
dadurch gekennzeichnet,
daß die flüssige Lösung bei einer Temperatur im Bereich von 100 bis 140 °C bevorratet wird.

3. Verfahren gemäß einem der vorhergehenden Ansprüche,
dadurch gekennzeichnet,
daß die Dicarbonsäure 6 bis 13 C-Atome im Kohlenstoffgerüst aufweist.

4. Verfahren gemäß einem der vorhergehenden Ansprüche,
dadurch gekennzeichnet,
daß es sich bei der Carbonsäure um Adipinsäure, Korksäure, Sebacinsäure, Azelainsäure, Undecandicarbonsäure, Dodecandicarbonsäure, dimerisierte Fettsäure, Brassylsäure, Cyclohexandicarbonsäure, Terephthalsäure, Isophthalsäure, 2,6-Naphthalindicarbonsäure und/oder 4,4'-Biphenyldicarbonsäure handelt.

5. Verfahren gemäß einem der vorhergehenden Ansprüche,
dadurch gekennzeichnet,
daß das Lactam der aliphatischen α, ω-Dicarbonsäure 6 bis 12 C-Atome im Kohlenstoffgerüst aufweist.

6. Verfahren gemäß einem der vorhergehenden Ansprüche,
dadurch gekennzeichnet,
daß es sich bei dem Lactam um Caprolactam, Laurinlactam, Capryllactam, Caprinlactam, Oenanthlactam und/oder Pelargonlactam handelt.

7. Verfahren gemäß einem der vorhergehenden Ansprüche,
dadurch gekennzeichnet,
daß die flüssige Lösung - bezogen auf die Lactammasse - bis zum 4-fachen an Dicarbonsäure enthält.

8. Verfahren gemäß einem der vorhergehenden Ansprüche,
dadurch gekennzeichnet,
daß die flüssige Lösung - bezogen auf die Lactammasse - bis zum 1,5-fachen an Dicarbonsäure enthält.

## Claims

1. A process for preparing copolyamides,
characterized in that
use is made for this purpose of the liquid solution of a dicarboxylic acid having from 6 to 44 carbon atoms in its carbon skeleton in a lactam of an aliphatic α,ω-aminocarboxylic acid having from 6 to 16 carbon atoms in its carbon skeleton, where the temperature at which the liquid solution is held available for use is in the range from 60 to 160°C.

2. A process according to claim 1,
characterized in that
the temperature at which the liquid solution is held available for use is in the range from 100 to 140°C.

3. A process according to any one of the preceding claims,
characterized in that
the dicarboxylic acid has from 6 to 13 carbon atoms in its carbon skeleton.

4. A process according to any one of the preceding claims,
characterized in that
the carboxylic acid is adipic acid, suberic acid, sebacic acid, azelaic acid, undecanedicarboxylic acid, dodecanedicarboxylic acid, dimerized fatty acid, brassilic acid, cyclohexanedicarboxylic acid, terephthalic acid, isophthalic acid, 2,6-naphthalenedicarboxylic acid and/or 4,4'-biphenyldicarboxylic acid.

5. A process according to any one of the preceding claims,
characterised in that
the lactam of the aliphatic α,ω-dicarboxylic acid has from 6 to 12 carbon atoms in its carbon skeleton.

6. A process according to any one of the preceding claims,
characterised in that
the lactam is caprolactam, laurolactam, capryllactam, capriclactam, oenantholactam and/or pelargonolactam.

7. A process according to any one of the preceding claims,
characterised in that
the liquid solution contains, based on the weight of the lactam, up to 4 times the amount of dicarboxylic acid.

8. A process according to any one of the preceding claims,
characterised in that
the liquid solution contains, based on the weight of the lactam, up to 1.5 times the amount of dicarboxylic acid.

## Revendications

1. Procédé de production de copolyamides,
caractérisé en ce qu'
on utilise à cet effet une solution liquide d'un acide dicarboxylique ayant de 6 à 44 atomes de carbone dans l'ossature carbonée, dans un lactame d'un acide α,ω-aminocarboxylique aliphatique ayant de 6 à 16 atomes de carbone dans l'ossature carbonée, la solution liquide étant introduite à une température comprise entre 60 et 160°C.

2. Procédé selon la revendication 1,
caractérisé en ce qu'
on introduit la solution liquide à une température comprise entre 100 et 140°C.

3. Procédé selon l'une des revendications précédentes,
caractérisé en ce que
l'acide dicarboxylique contient de 6 à 13 atomes de carbone dans l'ossature carbonée.

4. Procédé selon l'une des revendications précédentes,
caractérisé en ce que
l'acide carboxylique est l'acide adipique, l'acide subérique, l'acide sébacique, l'acide azélaïque, l'acide undécanedicarboxylique, l'acide dodécanedicarboxylique, un acide gras dimérisé, l'acide brassylique, l'acide cyclohexane dicarboxylique, l'acide téréphtalique, l'acide isophtalique, l'acide 2,6-naphtalène dicarboxylique et/ou l'acide 4,4'-biphényldicarboxylique.

5. Procédé selon l'une des revendications précédentes,
caractérisé en ce que
le lactame de l'acide α,ω-dicarboxylique aliphatique présente de 6 à 12 atomes de carbone dans l'ossature carbonée.

6. Procédé selon l'une des revendications précédentes,
caractérisé en ce que
le lactame est le caprolactame, le laurinelactame, le caprylactame, le caprinelactame, l'oenanthlactame et/ou le pelargonlactame.

7. Procédé selon l'une des revendications précédentes,
caractérisé en ce que
la solution liquide - par rapport à la masse de lactame - contient une quantité d'acide dicarboxylique jusqu'à quatre fois supérieure.

8. Procédé selon l'une des revendications précédentes,
caractérisé en ce que,
la solution liquide - par rapport à la masse de lactame - contient une quantité d'acide dicarboxylique jusqu'à 1,5 fois supérieure.
